# EUROPEAN PATENT SPECIFICATION

(11) **EP 3 713 999 B1**
(45) Date of publication and mention of the grant of the patent: **22.01.2025**
(21) Application number: 18822552.8
(22) Date of filing: 20.11.2018
(51) Int. Cl.: C08L 5/00, C12N 5/00, C12N 5/071, C12N 5/0775

(54) **DISSOLVABLE FOAM SCAFFOLDS FOR CELL CULTURE AND METHODS OF MAKING THE SAME**
LÖSLICHE SCHAUMGERÜSTE FÜR ZELLKULTUR UND VERFAHREN ZU DEREN HERSTELLUNG
ÉCHAFAUDAGES SOLUBLES DE MOUSSE POUR LA CULTURE CELLULAIRE ET PROCÉDÉS POUR LEUR FABRICATION

(30) Priority: 21.11.2017 US 201762589238 P; 19.02.2018 US 201862632178 P
(43) Date of publication of application: 30.09.2020
(73) Proprietor: Corning Incorporated, Corning, New York 14831 (US)
(72) Inventor: BEAR, Marie-Maud, 77590 Fontaine le port (FR); HENRY, David, 77590 Fontaine le Port (FR); HERVY, Martial, 77250 Veneux les sablons (FR); PÉCHEUL, Maryléne Denise Madeleine, 77460 Souppes Sur Loing (FR); WALERACK, Corinne, 77250 Veneux les sablons (FR)
(74) Representative: Elkington and Fife LLP
(86) International application number: PCT/US2018/062071
(87) International publication number: WO 2019/104069

(56) References cited:
- EP-A1- 0 044 624
- WO-A1-2005/023323
- WO-A1-2014/209865
- WO-A1-2016/200888
- US-A1- 2009 263 601
- ROBERTA GENTILINI ET AL: "Polysaccharide-based hydrogels with tunable composition as 3D cell culture systems", THE INTERNATIONAL JOURNAL OF ARTIFICIAL ORGANS, 19 February 2018 (2018-02-19), pages 213 - 222, XP055543864, Retrieved from the Internet <URL:https://journals.sagepub.com/doi/pdf/10.5301/ijao.5000667> [retrieved on 20190117]
- JAMES COURTENAY ET AL: "Recent Advances in Modified Cellulose for Tissue Culture Applications", MOLECULES, vol. 23, no. 3, 1 January 2018 (2018-01-01), DE, pages 654, XP055543865, ISSN: 1433-1373, DOI: 10.3390/molecules23030654
- NINAN NEETHU ET AL: "Pectin/carboxymethyl cellulose/microfibrillated cellulose composite scaffolds for tissue engineering", CARBOHYDRATE POLYMERS, vol. 98, no. 1, 7 July 2013 (2013-07-07), pages 877 - 885, XP028700413, ISSN: 0144-8617, DOI: 10.1016/J.CARBPOL.2013.06.067
- JI-SOO LEE ET AL: "Optimization of calcium pectinate gel beads for sustained-release of catechin using response surface methodology", INTERNATIONAL JOURNAL OF BIOLOGICAL MACROMOLECULES., vol. 42, no. 4, 18 January 2008 (2008-01-18), NL, pages 340 - 347, XP055543912, ISSN: 0141-8130, DOI: 10.1016/j.ijbiomac.2008.01.003

## Description

### CROSS REFERENCE TO RELATED APPLICATIONS

This application claims the benefit of priority of U.S. Provisional Application Serial No. 62/589,238 filed on November 21, 2017, and U.S. Provisional Application Serial No. 62/632,178 filed on February 19, 2018.

### FIELD

The present disclosure generally relates to dissolvable cell culture materials. In particular, the present disclosure relates to dissolvable foam scaffolds for cell culture and methods of making such dissolvable foam scaffolds.

### BACKGROUND

Vol. 98, no. 1, 07 July 2013, (2013-07-07), pages 877-885, XP028700413 discloses microfibrillated cellulose composite scaffolds for tissue engineering. WO 2005/023323 A1 discloses gelled biopolymer based foam. US2009263601 discloses articles prepared from biodegradable compositions and manufacturing methods. WO2014209865 discloses a cell culture article and methods thereof. WO2016200888 discloses digestible substrates for cell culture. International journal of biological macromolecules., NL, (20080118), vol. 42, no. 4 discloses optimization of calcium pectinate gel beads for sustained-release of catechin using response surface methodology.
In-vitro studies are important to the drug discovery process and exploration of potential new therapeutics. However, traditional in-vitro cell culture on two-dimensional (2D) culture substrates fails to simulate an in-vivo environment. Since almost all cells in the in-vivo environment are surrounded by other cells and extracellular matrix (ECM) in a three-dimensional (3D) fashion, 2D cell culture does not adequately simulate the natural 3D environment of cells. Cells in 2D culture are forced to adhere to a rigid surface and are geometrically constrained, adopting a flat morphology which alters the cytoskeleton regulation that is important in intracellular signaling, and consequently can affect cell growth, migration, and apoptosis. Moreover, organization of the ECM, which is significant to cell differentiation, proliferation, and gene expression, is absent in most 2D cells. These limitations of 2D cultures often result in biological responses in-vitro that are strikingly different from what is observed in-vivo.

Currently, in drug discovery, the standard procedure of screening compounds starts with 2D cell culture-based tests, followed by animal model tests, and then clinical trials. According to publicly available data, only about 10% of the compounds progress successfully through clinical development. Many drugs fail during clinical trials (especially during phase III, which is the most expensive phase of clinical development) largely due to the lack of clinical efficacy and/or unacceptable toxicity. A portion of these failures is attributed to data collected from the 2D culture tests in which the cellular response to drug(s) is altered due to their unnatural microenvironment. Due to the high costs associated with drug discovery, demand has risen for the ability to dismiss ineffective and/or unacceptable toxic compounds as early in the drug discovery process as possible. In-vitro cell-based systems that can more realistically mimic the in-vivo cell behaviors and provide more predictable results to in-vivo tests are presently being considered.

Recent research suggests that 3D cell culture, in contrast to 2D culture, more accurately represents the environment experienced by cells in-vivo, and it has been demonstrated that cell responses in 3D cultures are more similar to in-vivo behavior than the cell responses in 2D cultures. The additional dimensionality of 3D cultures is believed to lead to the differences in cellular responses because not only does it influence the spatial organization of the cell surface receptors engaged in interactions with surrounding cells, but it also induces physical constraints to cells. These spatial and physical aspects in 3D cultures are believed to affect the signal transduction from the outside to the inside of cells, and ultimately influence gene expression and cellular behavior.

The benefits of 3D culture have influenced the development of cell culture technologies which simulate the natural 3D environment of cells. Some bioreactors include a carrier in the form of a stationary packing material forming a fixed or packed bed for promoting cell adhesion and growth. The arrangement of the packing material of the fixed bed affects local fluid, heat, and mass transport, and usually is very dense to maximize cell cultivation in a given space. Yet another 3D cell culture technology is a porous 3D matrix or scaffold which promotes the growth and proliferation of the cultured cells within pores and other interior spaces of the matrix.

In each of the above described technologies, a protease treatment may be used to harvest the cells. However, commonly used harvesting procedures, such as protease treatment, subject the cells to harsh conditions which may damage cell structure and function. Additionally, protease treatment alone often causes only a limited amount of cell detachment. For the fixed bed material, the problem, in part, results from the densely packed nature of the fixed bed material which makes it more difficult to circulate the protease agent throughout the bed and increase the yield of cells harvested. Similarly, it can be difficult to circulate the protease agent through interior spaces of the 3D matrix, which in turn makes it difficult to dislodge cells during the harvest process. This difficulty is compounded by the presence of extracellular macromolecules secreted by the cultured cells that serve to attach the cells to the surface of the fixed bed material or to the surface of the matrix.

Either alternatively, or in combination with the protease treatment, methods and systems for harvesting cells have been developed that apply mechanical force to release cultured cells from the fixed bed material or the 3D matrix. For example, the fixed bed material or the 3D matrix, or a larger system including the fixed bed material or the 3D matrix, may be shaken or vibrated to release the cultured cells. Application of mechanical force may also cause physical damage to the cultured cells, which in turn reduces cell culture yields.

### SUMMARY

According to embodiments of the present disclosure, a dissolvable foam scaffold for cell culture is provided according to the present claims.

It is to be understood that both the foregoing general description and the following detailed description are merely exemplary, and are intended to provide an overview or framework to understanding the nature and character of the claims. The accompanying drawings are included to provide a further understanding, and are incorporated in and constitute a part of this specification. The drawings illustrate one or more embodiment(s), and together with the description serve to explain principles and operation of the various embodiments.

### BRIEF DESCRIPTION OF THE DRAWINGS

The disclosure will be understood more clearly from the following description and from the accompanying figures, given purely by way of non-limiting example, in which:
Figure 1 is a perspective view of a dissolvable foam scaffold in accordance with the present disclosure;
Figure 2 shows an SEM picture of a foam scaffold prepared in Example 1;
Figure 3 shows an SEM picture of a foam scaffold prepared in Example 2;
Figure 4 shows an SEM picture of a foam scaffold prepared in Example 3;
Figure 5 shows an SEM picture of a foam scaffold prepared in Example 4;
Figure 6 shows an SEM picture of a foam scaffold prepared in Example 5;
Figure 7 shows an SEM picture of a foam scaffold prepared in Example 6;
Figure 8 shows an SEM picture of a foam scaffold prepared in Example 7;
Figure 9 shows an SEM picture of a foam scaffold prepared in Example 8;
Figure 10 shows an SEM picture of a foam scaffold prepared in Example 9;
Figure 11 shows an SEM picture of a foam scaffold prepared in Example 10;
Figure 12 shows an SEM picture of a foam scaffold prepared in Example 11;
Figure 13 shows four foams prepared in Example 12 where varied amounts of a plasticizer were added to form the four foam scaffolds;
Figure 14 shows an SEM picture of a foam scaffold prepared in Example 13;
Figure 15 shows spheroids formed in the pores of a foam scaffold prepared in Example 4;
Figure 16 shows cells adhered to the foam scaffold prepared in Example 14;
Figure 17 shows cells adhered to the foam scaffold prepared in Example 15;
Figure 18 shows cells after six days of expansion adhered to the foam scaffold prepared in Example 15;
Figure 19 is a bar graph showing the GFP-positive percentage of transfected HEK cells for each of the culture conditions of Example 24;
Figure 20 is a bar graph showing GFP-positive percentage of transfected cells per set of foam scaffold of Example 25;
Figure 21 is a bar graph showing the number of viral particles (vp) per set of foam scaffold of Example 25;
Figure 22 is a bar graph showing the number of viral particles per cell obtained per set of foam scaffold of Example 25; and
Figure 23 is a bar graph showing the fraction of cells exhibiting GFP expression for each of the sets of foam scaffolds of Example 25 after infection.

### DETAILED DESCRIPTION

Reference will now be made in detail to the present embodiment(s), an example(s) of which is/are illustrated in the accompanying drawings. Whenever possible, the same reference numerals will be used throughout the drawings to refer to the same or like parts.

The singular forms "a," "an" and "the" include plural referents unless the context clearly dictates otherwise. The endpoints of all ranges reciting the same characteristic are independently combinable and inclusive of the recited endpoint.

As used herein, "have," "having," "include," "including," "comprise," "comprising" or the like are used in their open ended sense, and generally mean "including, but not limited to."

All scientific and technical terms used herein have meanings commonly used in the art unless otherwise specified. The definitions provided herein are to facilitate understanding of certain terms used frequently herein and are not meant to limit the scope of the present disclosure.

The present disclosure is described below, at first generally, then in detail on the basis of several exemplary embodiments. The features shown in combination with one another in the individual exemplary embodiments do not all have to be realized. In particular, individual features may also be omitted or combined in some other way with other features shown of the same exemplary embodiment or else of other exemplary embodiments.

Embodiments of the present disclosure relate to dissolvable foam scaffolds for cell culture and methods of making such dissolvable foam scaffolds. Embodiments of the present disclosure further relate to methods of cell culture of adherent cells, cell aggregates, or spheroids, in dissolvable foam scaffolds. Furthermore, embodiments of the present disclosure relate to bioreactors systems including dissolvable foam scaffolds. As will become clearer in the discussions below, foam scaffolds as disclosed herein are described as being dissolvable and insoluble. As used herein, the term "insoluble" is used to refer to a material or combination of materials that is not soluble, and that remains crosslinked, under conventional cell culture conditions which include, for example, cell culture media. Also as used herein, the term "dissolvable" is used to refer to a material or combination of materials that is digested when exposed to an appropriate concentration of an enzyme that digests or breakdowns the material or combination of materials. Dissolvable foam scaffolds as described herein are porous scaffolds having an open pore architecture and highly interconnected pores. The pores of the scaffolds provide a protected environment for the culturing of cells where the cell-to-cell interactions and formation of ECM in a 3D fashion are aided. The dissolvable foam scaffolds may be completely digested which allows for harvesting cells without damaging the cells using protease treatment and/or mechanical harvesting techniques.

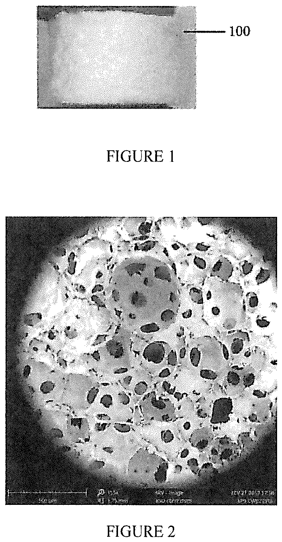

Figure 1 is a perspective view of a dissolvable foam scaffold **10** in accordance with the present disclosure. As will be described in further detail below and as will become more clear from the other figures of the present disclosure, dissolvable foam scaffold **10** is a porous foam that includes an open pore architecture. Dissolvable foam scaffold **10** has a porosity of from about 85% to about 96% and an average pore size diameter of between about 50 µm and about 500 µm. Dissolvable foam scaffold **10** provides a protected environment within the pores of the foam scaffold for the culturing of cells. Additionally, dissolvable foam scaffold **10** is also dissolvable when exposed to an appropriate enzyme that digests or breakdowns the material which facilitates harvesting of the cells cultured in the scaffold without damaging the cells.

Dissolvable foam scaffolds as described herein include at least one ionotropically crosslinked polysaccharide. Generally, polysaccharides possess attributes beneficial to cell culture applications. Polysaccharides are hydrophillic, non-cytotoxic and stable in culture medium. Examples include pectic acid, also known as polygalacturonic acid (PGA), or salts thereof, partly esterified pectic acid or salts thereof, or partly amidated pectic acid or salts thereof. Pectic acid can be formed via hydrolysis of certain pectin esters. Pectins are cell wall polysaccharides and in nature have a structural role in plants. Major sources of pectin include citrus peel (e.g., peels from lemons and limes) and apple peel. Pectins are predominantly linear polymers based on a 1,4-linked alpha-D-galacturonate backbone, interrupted randomly by 1,2-linked L-rhamnose. The average molecular weight ranges from about 50,000 to about 200,000 Daltons.

The polygalacturonic acid chain of pectin may be partly esterified, e.g., methyl groups and the free acid groups may be partly or fully neutralized with monovalent ions such as sodium, potassium, or ammonium ions. Polygalacturonic acids partly esterified with methanol are called pectinic acids, and salts thereof are called pectinates. The degree of methylation (DM) for high methoxyl (HM) pectins can be, for example, from 60 to 75 mol% and those for low methoxyl (LM) pectins can be from 1 to 40 mol%. The degree of esterification of partly esterified polygalacturonic acids as described herein may be less than about 70 mol%, or less than about 60 mol%, or less than 50 mol%, or even less than about 40 mol%, and all values therebetween. Without wishing to be bound by any particular theory, it is believed that a minimum amount of free carboxylic acid groups (not esterified) facilitates a degree of ionotropic crosslinking which allow for the formation of a dissolvable scaffold which is insoluble.

Alternatively, the polygalacturonic acid chain of pectin may be partly amidated. Polygalacturonic acids partly amidated pectin may be produced, for example, by treatment with ammonia. Amidated pectin contains carboxyl groups (~COOH), methyl ester groups (~COOCH₃), and amidated groups (-CONH₂). The degree of amidation may vary and may be, for example, from about 10% to about 40% amidated.

According to embodiments of the present disclosure, dissolvable foam scaffolds as described herein may include a mixture of pectic acid and partly esterified pectic acid. Blends with compatible polymers may also be used. For example, pectic acid and/or partly esterified pectic acid may be mixed with other polysaccharides such as dextran, substituted cellulose derivativdes, alginic acid, starches, glycogen, arabinoxylans, agarose, etc. Glycosaminoglycans like hyaluronic acid and chondroitin sulfate, or various proteins such as elastin, fibrin, silk fibroin, collagen and their derivatives can be also used. Water soluble synthetic polymers can be also blended with pectic acid and/or partly esterified pectic acid. Exemplary water soluble synthetic polymers include, but are not limited to, polyalkylene glycol, poly(hydroxyalkyl(meth)acrylates), poly(meth)acrylamide and derivatives, poly(N-vinyl-2-pyrrolidone), and polyvinyl alcohol.

According to embodiments of the present disclosure, dissolvable foam scaffolds as described herein includes at least one first polymer. The at least one first polymer is water soluble, non-ionotropically crosslinkable and has surface activity. As used herein, the term "surface activity" refers to the activity of an agent to lower or eliminate the surface tension (or interfacial tension) between two liquids or between a liquid and a solid or between gas and liquid. The at least one first polymer may have a hydrophilic-lipophilic balance (HLB) of greater than about 8 or even greater than about 10. For example, the at least one first polymer may have an HLB of between about 8 and about 40 or between about 10 and about 40. The at least one first polymer may have an HLB of between about 8 and about 15, or even between about 10 and about 12. HLB provides a reference for the lipophilic or hydrophilic degree of a polymer. A larger HLB value indicates stronger hydrophilicity, while a smaller HLB value indicates a stronger lipophilicity. In general, the HLB value varies in the range of from 1 to 40 and the hydrophilic-lipophilic transition is often considered to be between about 8 and about 10. When the HLB value is less than the hydrophilic-lipophilic transition, the material is lipophilic, and when the HLB value is greater than the hydrophilic-lipophilic transition the material is hydrophilic.

Exemplary first polymers are in accordance with the present claims. Exemplary cellulose derivatives include, but are not limited to, hydroxyethylcellulose (HEC), hydroxypropylcellulose (HPC), methylcellulose (MC), hydroxyethylmethylcellulose (HEMC), and hydroxypropyl-methylcellulose (HPMC). Exemplary proteins include, but are not limited to, bovine serum albumin (BSA), gelatine, casein and hydrophobins. Exemplary synthetic amphiphilic polymers include, but are not limited to, a poloxamer available under the trade name Synperonics^{®} (commercially available from Croda International, Snaith, United Kingdom), a poloxamer available under the trade name Pluronics^{®} (commercially available from BASF Corp., Parsippany, NJ) and a poloxamer available under the trade name Kolliphor^{®} (commercially available from BASF Corp., Parsippany, NJ).

Dissolvable foam scaffolds as described herein may include at least one second polymer comprising dextrin. The at least one second polymer is water soluble and has no surface activity. Exemplary second polymers may be any of synthetic polymers, semisynthetic polymers, natural polymers and combinations thereof. Exemplary synthetic polymers include, but are not limited to, polyethylene glycol, polyvinyl pyrrolidone, polyvinyl alcohol, carboxyvinyl polymer, polyacrylic acid, polyacrylamide, homopolymer and copolymer of N-(2-Hydroxypropyl) methacrylamide, polyvinyl methyl ether-maleic anhydride, and polyethylene oxide/polypropylene oxide block copolymers. Exemplary semisynthetic polymers include, but are not limited to, dextran derivatives, carboxymethyl cellulose, hydroxyethyl cellulose and derivatives, methylcellulose and derivatives, ethylcellulose cellulose, ethyl hydroxyethyl cellulose, and hydroxypropyl cellulose. Exemplary natural polymers include, but are not limited to, starch and starch derivatives, polymers obtained by microbial fermentation such as curdlan, pullulan and gellan gum, xanthan gum, dextran, proteins such as albumin, casein and caseinates, gelatin, seaweed extracts such as agar, alginates and carrageenan, seed extracts such as guar gum and derivatives and locust bean gum, hyaluronic acid, and chondroitin sulfate.

Dissolvable foam scaffolds as described herein may be crosslinked to increase their mechanical strength and to prevent the dissolution of the scaffolds when placed in contact with cell culture medium. Crosslinking may be performed by ionotropic gelation as described below wherein ionotropic gelation is based on the ability of polyelectrolytes to crosslink in the presence of multivalent counter ions to form crosslinked scaffolds. Without wishing to be bound by any particular theory, it is believed that ionotropic gelation of the polysaccharide of the dissolvable foam scaffolds is the result of strong interactions between divalent cations and the polysaccharide.

According to embodiments of the present disclosure, scaffolds as described herein are porous foam scaffolds. Foam scaffolds as described herein may have a porosity of from about 85% to about 96%. For example, foam scaffolds as described herein may have a porosity of from about 91% to about 95%, or about 94% to about 96%. As used herein, the term "porosity" refers to the measure of open pore volume in the dissolvable scaffold and is referred to in terms of % porosity, wherein % porosity is the percent of voids in the total volume of the dissolvable foam scaffold. Foam scaffolds as described herein may have an average pore size diameter of between about 50 µm and about 500 µm. For example, average pore size diameter may be between about 75 µm and about 450 µm, or between about 100 µm and about 400 µm, or even between 150 µm and about 350 µm and all values therebetween.

Scaffolds as described herein may have a wet density of less than about 0.40 g/cc. For example, scaffolds as described herein may have a wet density of less than about 0.35 g/cc, or less than about 0.30 g/cc, or less than about 0.25 g/cc. Scaffolds as described herein may have a wet density of between about 0.16 g/cc and about 0.40 g/cc, or between about 0.16 g/cc and about 0.35 g/cc, or between about 0.16 g/cc and about 0.30 g/cc, or even between about 0.16 g/cc and about 0.25 g/cc, and all values therebetween. Scaffolds as described herein have a dry density of less than 0.11 g/cc, or less than about 0.10 g/cc, or less than about 0.05 g/cc. Scaffolds as described herein may have a dry density of between about 0.02 g/cc and about 0.10 g/cc, or even between about 0.02 g/cc and about 0.05 g/cc, and all values therebetween.

Several pore types are possible in scaffolds. Open pores allow for cellular access on both sides of the scaffold and allow for liquid flow and transport of nutrients through the dissolvable scaffold. Partially open pores allow for cellular access on one side of the scaffold, but mass transport of nutrients and waste products is limited to diffusion. Closed pores have no openings and are not accessible by cells or by mass transport of nutrients and waste products. Dissolvable foam scaffolds as described herein have an open pore architecture and highly interconnected pores. Generally, the open pore architecture and highly interconnected pores enable migration of cells into the pores of the dissolvable foam scaffolds and also facilitate enhanced mass transport of nutrients, oxygen and waste products. The open pore architecture also influences cell adhesion and cell migration by providing a high surface area for cell-to-cell interactions and space for ECM regeneration.

Dissolvable foam scaffolds as described herein are digested when exposed to an appropriate enzyme that digests or breakdowns the material. Non-proteolytic enzymes suitable for digesting the foam scaffolds, harvesting cells, or both, include pectinolytic enzymes or pectinases, which are a heterogeneous group of related enzymes that hydrolyze the pectic substances. Pectinases (polygalacturonase) are enzymes that break down complex pectin molecules to shorter molecules of galacturonic acid. Commercially available sources of pectinases are generally multi-enzymatic, such as Pectinex^{™} ULTRA SP-L (commercially available from Novozyme North American, Inc., Franklinton, NC), a pectolytic enzyme preparation produced from a selected strain of Aspergillus aculeatus. Pectinex^{™} ULTRA SP-L contains mainly polygalacturonase, (EC 3.2.1.15) pectintranseliminase (EC 4.2.2.2) and pectinesterase (EC: 3.1.1.11). The EC designation is the Enzyme Commission classification scheme for enzymes based on the chemical reactions the enzymes catalyze.

According to embodiments of the present disclosure, digestion of the dissolvable foam scaffolds also includes exposing the scaffold to a divalent cation chelating agent. Exemplary chelating agents include, but are not limited to, ethylenediaminetetraacetic acid (EDTA), cyclohexanediaminetetraacetic (CDTA), ethylene glycol tetraacetic acid (EGTA), citric acid and tartaric acid.

The time to complete digestion of dissolvable foam scaffolds as described herein may be less than about 1 hour. For example, the time to complete digestion of foam scaffolds may be less than about 45 minutes, or less than about 30 minutes, or less than about 15 minutes, or between about 1 minute and about 25 minutes, or between about 3 minutes and about 20 minutes, or even between about 5 minutes and about 15 minutes.

According to embodiments of the present disclosure, scaffolds as described herein may further include an adhesion polymer coating. The adhesion polymer may include peptides. Exemplary peptides may include, but are not limited to BSP, vitronectin, fibronectin, laminin, Type I and IV collagen, denatured collagen (gelatin), and like peptides, and mixtures thereof. Additionally, the peptides may be those having an RGD sequence. The coating may be, for example, Synthemax^{®} II-SC (commercially available from Corning, Incorporated, Corning, NY). Optionally, the adhesion polymer may include an extracellular matrix. The coating may be, for example, Matrigel^{®} (commercially available from Corning, Incorporated, Corning, NY).

According to embodiments of the present disclosure, methods for forming dissolvable foam scaffolds as described herein are also disclosed. Methods as described herein may include forming a first aqueous mixture which includes dissolving a polysaccharide in an aqueous solution. Polysaccharides may be those as described above, such as pectic acid or salts thereof, partly esterified pectic acid or salts thereof, or partly amidated pectic acid or salts thereof, and blends of such polysaccharides.

Methods for forming dissolvable foam scaffolds as described herein may further include forming a second aqueous mixture including a water insoluble divalent metal salt in an aqueous solution. Metals of the divalent metal salts may include, but are not limited to, magnesium, calcium, zinc, strontium, barium, and like cations, and combinations thereof. Anions of the divalent metal salts may include, but are not limited to, oxalates, tartrates, phosphates, carbonates, citrates, and like organic and inorganic anions, and combinations thereof.

According to embodiments of the present disclosure, forming a second aqueous mixture may further include adding the at least one first polymer as described above to the second aqueous mixture. Optionally, methods as described herein may further include adding the at least one second polymer as described above to the second aqueous mixture. According to embodiments of the present disclosure, the at least one first polymer and the at least one second polymer may be added to the second aqueous mixture separately or may be added to the second aqueous mixture together. When added as a mixture, the mixture may include about 50% of the at least one first polymer and about 50% of the at least one second polymer. For example, the mixture may include between about 35% and about 65% (and all values therebetween) of the at least one first polymer and between about 35% and about 65% (and all values therebetween) of the at least one second polymer.

According to embodiments of the present disclosure, forming a second aqueous mixture may further include adding a water soluble plasticizer to the second aqueous mixture. Plasticizers as described herein are non-toxic and do not affect the solubility of the polysaccharides of the dissolvable foam scaffolds. A plasticizer provides flexibility and softness to the resulting foam such that the resulting foam is soft and pliable. Plasticizers as described herein may include, but are not limited to, polyhydric alcohols such as glycerol, sorbitol, ethylene glycol, propylene glycol, polyethylene glycol and combinations thereof. Adding a water soluble plasticizer to the second aqueous mixture includes adding between about 15 wt. % and about 55 wt. % ,or between about 15 wt. % and about 50 wt. %, or between about 15 wt. % and about 40 wt. %, or between about 15 wt. % and about 30 wt. %, or between about 15 wt. % and about 25 wt. % of the total solid additives added to form the second aqueous mixture, and all values therebetween. As used herein, the term "total solid additives added to form the second aqueous mixture" refers to all of the components of the aqueous mixture except for water.

According to embodiments of the present disclosure, forming a second aqueous mixture may further include adding an emulsifying agent to the second aqueous mixture. Emulsifying agents as described herein may include, but are not limited to, Tween^{®} 20, Tween^{®} 80 (each commercially available from commercially available from Croda International, Snaith, United Kingdom).

According to embodiments of the present disclosure, forming a second aqueous mixture may further include adding at least one leachable solid to the second aqueous mixture. Leachable solids as described herein include materials that reinforce or create pores during the formation of the foam scaffold. Leachable solids may be, but are not limited to, nontoxic leachable materials such as salts, biocompatible mono and disaccharides and water-soluble proteins. Exemplary salts include, but are not limited to, sodium chloride, potassium chloride, calcium chloride, sodium tartrate, sodium citrate, and the like. Exemplary biocompatible mono and disaccharides include, but are not limited to, glucose, fructose, dextrose, maltose, lactose and sucrose. Exemplary water-soluble proteins include, but are not limited to, gelatin and agarose.

Each of the materials described above in relation to the second aqueous mixture may all be optionally added to the second aqueous mixture and may be added to the second aqueous mixture in any order with the possibility that two or more of the materials may be added to the second aqueous mixture simultaneously. In one exemplary method, forming a second aqueous mixture includes adding a leachable solid to an aqueous solution including a divalent metal salt and mixing the aqueous mixture to facilitate the dissolution of the leachable solid dissolves in the aqueous mixture. The at least one first polymer, the at least one second polymer and/or the water soluble plasticizer are subsequently added to the second aqueous mixture.

Methods for forming a dissolvable foam scaffold as described herein further include, subsequent to forming the first and second aqueous mixtures, combining the second aqueous mixture with the first aqueous mixture to form a combined aqueous mixture. A foam may be formed from the combined aqueous mixture by introducing gas bubbles into the aqueous mixture through mixing, beating, agitating, aerating, whipping, injecting or other mechanical actions. The gas may be for example, but not limited to, air, nitrogen, helium, hydrogen, argon, carbon dioxide or other inert gas. Introducing gas bubbles into the combined aqueous mixture may then be done for a period of less than about 30 minutes, for example, between about 1 minute and about 30 minutes, or between about 3 minutes and about 25 minutes, or even between about 5 minutes and about 20 minutes. While introducing gas bubbles into the combined aqueous mixture, the method for forming a dissolvable foam scaffold may further include adding a gel inducing agent to the combined aqueous mixture. The gel inducing agent may be an acid that provides a buffering action and/or materials that slowly generate acid. Exemplary acids include, but are not limited to, lactic acid lactone, glycolic acid lactone, glucono delta lactone and acid anhydrides.

Methods for forming a dissolvable foam scaffold as described herein may further include, coating the dissolvable foam scaffold with an adhesion polymer coating. Coating the dissolvable foam scaffold may include exposing the scaffold to an aqueous solution having an adhesion polymer in the aqueous solution. As previously discussed, the adhesion polymer may include peptides. Exemplary peptides may include, but are not limited to BSP, vitronectin, fibronectin, laminin, Type I and IV collagen, denatured collagen (gelatin), and like peptides, and mixtures thereof. Additionally, the peptides may be those having an RGD sequence. The coating may be, for example, Synthemax^{®} II-SC (commercially available from Corning, Incorporated, Corning, NY).

According to embodiments of the present disclosure, methods for culturing cells on dissolvable foam scaffolds as described herein are also disclosed. Any type of cell may be cultured on the dissolvable foam scaffolds including, but not limited to, immortalized cells, primary culture cells, cancer cells, stem cells (e.g., embryonic or induced pluripotent), etc. The cells may be mammalian cells, avian cells, piscine cells, etc. The cells may be of any tissue type including, but not limited to, kidney, fibroblast, breast, skin, brain, ovary, lung, bone, nerve, muscle, cardiac, colorectal, pancreas, immune (e.g., B cell), blood, etc. The cells may be in any cultured form in the bag including disperse (e.g., freshly seeded), confluent, 2-dimensional, 3-dimensional, spheroid, etc. Culturing cells on a dissolvable foam scaffold may include seeding cells on the dissolvable foam scaffold. Seeding cells on a dissolvable foam scaffold may include contacting the scaffold with a solution containing the cells. During seeding cells on the dissolvable foam scaffold, the cells enter the pores of the dissolvable foam scaffold. Where the dissolvable foam scaffold includes an adhesion polymer coating, cells may enter the pores of the dissolvable foam scaffold and attach to the scaffold material.

Culturing cells on dissolvable foam scaffolds may further include contacting the scaffolds with cell culture medium. Generally, contacting the scaffolds with cell culture medium includes placing cells to be cultured on the scaffolds in an environment with medium in which the cells are to be cultured. Contacting the scaffolds with cell culture medium may include pipetting cell culture medium onto the scaffolds, or submerging the scaffolds in cell culture medium, or passing cell culture media over the scaffolds in a continuous manner. Generally, as used herein, the term "continuous" refers to culturing cells with a consistent flow of cell culture medium into and out of the cell culture environment. Such passing cell culture media over the scaffolds in a continuous manner may include submerging the scaffolds in cell culture medium for a predetermined period of time, then removing at least some of the cell culture medium after the predetermined period of time and adding fresh cell culture medium such that the volume of cell culture medium in contact with the dissolvable foam scaffold remains substantially constant. Cell culture medium may be removed and replaced according to any predetermined schedule. For example, at least some of the cell culture medium may be removed and replaced every hour, or every 12 hours, or every 24 hours, or every 2 days, or every 3 days, or every 4 days, or every 5 days.

Cell culture medium may be for example, but is not limited to, sugars, salts, amino acids, serum (e.g., fetal bovine serum), antibiotics, growth factors, differentiation factors, colorant, or other desired factors. Exemplary cell culture medium includes Dulbecco's Modified Eagle Medium (DMEM), Ham's F12 Nutrient Mixture, Minimum Essential Media (MEM), RPMI Medium, Iscove's Modified Dulbecco's Media (IMDM) Mesencult^{™}-XF medium, and the like.

According to embodiments of the present disclosure, methods for harvesting cells from dissolvable foam scaffolds as described herein are also disclosed. Methods for harvesting cells as described herein may include digesting the dissolvable foam scaffold by exposing the dissolvable foam scaffold to an enzyme. As previously discussed, non-proteolytic enzymes suitable for digesting the foam scaffolds, harvesting cells, or both, include pectinolytic enzymes or pectinases, which are a heterogeneous group of related enzymes that hydrolyze the pectic substances. Commercially available sources of pectinases are generally multi-enzymatic, such as Pectinex^{™} ULTRA SP-L (commercially available from Novozyme North American, Inc., Franklinton, NC), a pectolytic enzyme preparation produced from a selected strain of Aspergillus aculeatus. Pectinex^{™} ULTRA SP-L contains mainly polygalacturonase, (EC 3.2.1.15) pectintranseliminase (EC 4.2.2.2) and pectinesterase (EC: 3.1.1.11). The EC designation is the Enzyme Commission classification scheme for enzymes based on the chemical reactions the enzymes catalyze.

Exposing the dissolvable foam scaffold to an enzyme may include exposing the scaffold to enzyme concentrations of between about 1 and about 200 U. For example, the method may include exposing the scaffold to enzyme concentrations of between about 2 U and about 150 U, or between about 5 U and about 100 U, or even between about 10 U and about 75 U, and all values therebetween.

Methods for harvesting cells as described herein may further include exposing the material to a chelating agent. Exemplary chelating agents include, but are not limited to, ethylenediaminetetraacetic acid (EDTA), cyclohexanediaminetetraacetic (CDTA), ethylene glycol tetraacetic acid (EGTA), citric acid and tartaric acid. Exposing the dissolvable foam scaffold to a chelating agent may include exposing the scaffold to chelating agent concentrations of between about 1 mM and about 200 mM. For example, the method may include exposing the scaffold to chelating agent concentrations of between about 10 mM and about 150 mM, or between about 20 mM and about 100 mM, or even between about 25 mM and about 50 mM, and all values therebetween.

### EXAMPLES

Embodiments of the present disclosure are further described below with respect to certain exemplary and specific embodiments thereof, which are illustrative only and not intended to be limiting. Examples 1-8 do not comprise a second polymer comprising dextrin therefore are not covered by the scope of the claims. It is further noted that examples wherein the dissolvable foam scaffold does not have a dry density of less than 0.11 g/cc are not covered by the claims.

### Example 1

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Methocel HPMC Culminal 724 | 1.94 grams |
| Non-Surface Active Polymer | None | |
| Gel Inducing Agent | GDL | 3.77 grams |

A first aqueous mixture containing 2.0 wt. % polygalacturonic acid (PGA) was prepared by dissolving about 162 grams of polygalacturonic acid sodium salt in demineralized water in an oil bath set at a temperature of 104°C. The aqueous mixture was cooled to room temperature. A second aqueous mixture was prepared by adding about 1.06 grams of CaCO₃ to about 24.52 grams of ultrapure water in the bowl of a KitchenAid mixer equipped with a wire loop whip. About 0.125 grams of TWEEN^{®} 20 (commercially available from Sigma-Aldrich, St. Louis, MO) was also added to the bowl of the KitchenAid mixer. About 17.5 grams of sucrose was then added to the mixer bowl and mixed to facilitate dissolution of the sucrose in the second aqueous mixture. About 7.5 grams glycerol, about 1.94 grams Methocel HPMC Culminal 724 and the first aqueous mixture were added to form a combined aqueous mixture in the mixing bowl and mixed at a stir speed (speed 1 of the KitchenAid mixer) for about 5 minutes. The combined aqueous mixture was then whipped at a fast whipping speed (speed 10 of the KitchenAid mixer) for about 20 minutes to introduce air into the combined aqueous mixture. While continuing to whip the combined aqueous mixture, a solution of about 3.77 grams of gluconolactone (GDL) in about 30 mL of water was added to the mixing bowl and whipping was continued for about 1 minute.

An opaque white foam having a wet foam density of about 0.25 g/cc was obtained following the process discussed above. The foam was left uncovered in the mixing bowl at room temperature for about 1 hour to allow time for crosslinking to take place within the foam. The foam was then exposed to temperatures of about -80°C for about 16 hours to freeze the foam and then exposed to a temperature of -86°C and a pressure of 0.11 mbar for about 72 hours. The resulting foam was observed to have a dry foam density of about 0.04 to about 0.045 g/cc and was observed to be porous with highly interconnected pores. Figure 2 shows an SEM picture of the foam prepared in this Example 1. The composition of the resulting foam was determined to include about 1.3 wt. % PGA, about 0.78 wt. % HPM and a total solid content of about 2.08 wt. %.

### Example 2

| **Additive Component** | **Type of Additive** | **Amount of Additive** |
|---|---|---|
| Polysaccharide | 2.0 wt. % PGA | 162 grams |
| Divalent Metal Salt | CaCO₃ | 0.53 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Methocel HPMC Culminal 724 | 1.94 grams |
| Non-Surface Active Polymer | None | |
| Gel Inducing Agent | GDL | 3.77 grams |

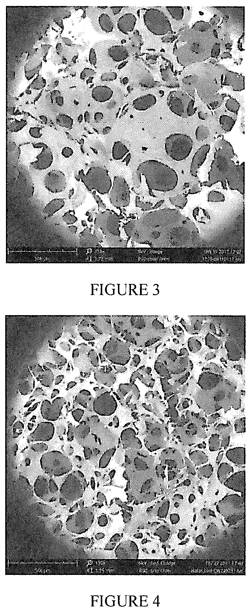

The process as described in Example 1 was repeated except the second aqueous mixture was prepared by adding about 0.53 grams. The resulting foam was observed to be porous with highly interconnected pores. Figure 3 shows an SEM picture of the foam prepared in this Example 2.

### Example 3

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Methocel HPMC Culminal 724 | 1.94 grams |
| Surface Active Polymer | Bovine Gelatin | 0.97 grams |
| Non-Surface Active Polymer | None | |
| Gel Inducing Agent | GDL | 3.77 grams |

A first aqueous mixture containing 2.0 wt. % polygalacturonic acid (PGA) was prepared by dissolving about 162 grams of polygalacturonic acid sodium salt in demineralized water in an oil bath set at a temperature of 104°C. The aqueous mixture was cooled to room temperature. A second aqueous mixture was prepared by adding about 7.5 grams of glycerol to ultrapure water and heating under a microwave at 800W for about 30 seconds. About 0.97 grams of bovine gelatin was cold-swelled in about 5.8 mL of ultrapure water and then added to the second aqueous mixture and stirred until dissolution was observed. About 1.06 grams of CaCO₃ and about 0.125 grams of TWEEN^{®} 20 were added to the second aqueous mixture. The second aqueous mixture was then sonicated for about 1 minute and then transferred to the bowl of a KitchenAid mixer equipped with a wire loop whip. About 17.5 grams sucrose and about 0.97 grams Methocel HPMC Culminal 724 were then added to the bowl of the KitchenAid mixer and the aqueous mixture was stirred for about 5 minutes. The first aqueous mixture containing 2.0 wt. % PGA were added to form a combined aqueous mixture in the mixing bowl and mixed at a stir speed (speed 1 of the KitchenAid mixer) for about 3 minutes. The combined aqueous mixture was then whipped at a fast whipping speed (speed 10 of the KitchenAid mixer) for about 20 minutes to introduce air into the combined aqueous mixture. While continuing to whip the combined aqueous mixture, a solution of about 3.77 grams of gluconolactone (GDL) in about 30 mL of water was added to the mixing bowl and whipping was continued for about 1 minute.

An opaque white foam was obtained following the process discussed above. The foam was left uncovered in the mixing bowl at room temperature for about 1 hour to allow time for crosslinking to take place within the foam. The foam was then exposed to temperatures of about -80°C for about 16 hours to freeze the foam and then exposed to a temperature of -86°C and a pressure of 0.11 mbar for about 72 hours. The resulting foam was observed to have a dry foam density of about 0.04 g/cc and was observed to be porous with highly interconnected pores. Figure 4 shows an SEM picture of the foam prepared in this Example 3.

### Example 4

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Methocel HPMC Culminal 724 | 1.94 grams |
| Surface Active Polymer | Porcine Gelatin | 0.97 grams |
| Non-Surface Active Polymer | None | |
| Gel Inducing Agent | GDL | 3.77 grams |

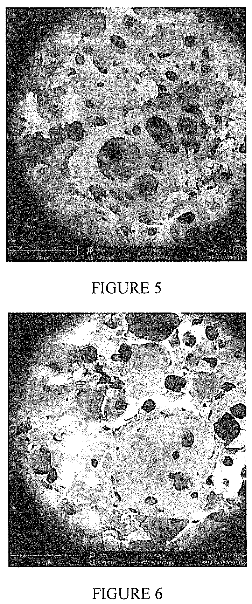

The process as described in Example 3 was repeated except that about 0.97 grams of porcine gelatin, instead of bovine gelatin, was cold-swelled in about 5.8 mL of ultrapure water and then added to the second aqueous mixture and stirred until dissolution was observed. The resulting foam was observed to have a wet foam density of about 0.21 g/cc and a dry foam density of about 0.06 g/cc and was observed to be porous with highly interconnected pores. Figure 5 shows an SEM picture of the foam prepared in this Example 4.

### Example 5

| **Additive Component** | **Type of Additive** | **Amount of Additive** |
|---|---|---|
| Polysaccharide | 3.0 wt. % PGA | 172 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | None | |
| Non-Surface Active Polymer | None | |
| Gel Inducing Agent | GDL | 3.77 grams |

The process as described in Example 1 was repeated except the first aqueous mixture was prepared by dissolving about 172 grams of 3.0 wt. % polygalacturonic acid sodium salt in demineralized water in an oil bath set at a temperature of 104°C. Additionally, Methocel HPMC Culminal 724 was omitted from the second aqueous mixture. The resulting foam was observed to have a wet foam density of about 0.40 g/cc and a dry foam density of about 0.11 g/cc. The foam was observed to be less porous than the foam formed in Example 1 and the pores were less interconnected than the pores of the foam formed in Example 1. It is believed that the omission of Methocel HPMC Culminal 724, or any polymer having surface activity, reduced the porosity and the level of interconnectivity between the pores of the foam. Figure 6 shows an SEM picture of the foam prepared in this Example 5. Although a foam having a wet density of greater than about 0.40 g/cc and a dry density of greater than about 0.11 g/cc was demonstrated as being able to support culturing of cells, foams having a wet density of less than about 0.40 g/cc and a dry density of less than about 0.11 g/cc exhibited improved cell culturing conditions as compared to foams having wet and dry densities such as those possessed by the foam as formed in this Example 5.

### Example 6

| **Additive Component** | **Type of Additive** | **Amount of Additive** |
|---|---|---|
| Polysaccharide | 1.59 wt. % PGA | 162 grams |
| Divalent Metal Salt | CaCO₃ | 0.53 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Methocel HPMC Culminal 724 | 2.58 grams |
| Non-Surface Active Polymer | None | |
| Gel Inducing Agent | GDL | 3.77 grams |

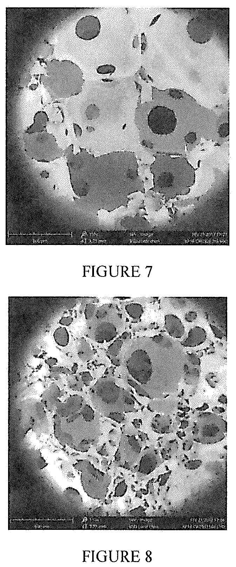

The process as described in Example 1 was repeated except the first aqueous mixture was prepared by dissolving about 162 grams of 1.59 wt. % polygalacturonic acid sodium salt in demineralized water in an oil bath set at a temperature of 104°C. Additionally, the second aqueous mixture was prepared by adding 0.53 grams of CaCO₃ to about 24.52 grams of ultrapure water and about 2.58 grams Methocel HPMC Culminal 724 was added to the second aqueous mixture. The resulting foam was observed to have a wet foam density of about 0.36 g/cc and a dry foam density of about 0.09 g/cc. Figure 7 shows an SEM picture of the foam prepared in this Example 6.

### Example 7

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | None | |
| Surface Active Polymer | Methocel HPMC Culminal 724 | 1.94 grams |
| Non-Surface Active Polymer | None | |
| Gel Inducing Agent | GDL | 3.77 grams |

The process as described in Example 1 was repeated except that sucrose was not added to the second aqueous mixture. The resulting foam was observed to have a wet foam density of about 0.23 g/cc and a dry foam density of about 0.03 g/cc. Figure 8 shows an SEM picture of the foam prepared in this Example 7.

### Example 8

| **Additive Component** | **Type of Additive** | **Amount of Additive** |
|---|---|---|
| Polysaccharide | 2.59 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Methocel HPMC Culminal 724 | 0.97 grams |
| Non-Surface Active Polymer | None | |
| Gel Inducing Agent | GDL | 3.77 grams |

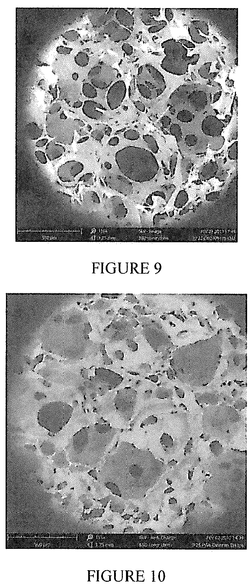

The process as described in Example 1 was repeated except the first aqueous mixture was prepared by dissolving about 162 grams of 2.59 wt. % polygalacturonic acid sodium salt in demineralized water in an oil bath set at a temperature of 104°C. Additionally, the second aqueous mixture was prepared by adding 0.97 grams Methocel HPMC Culminal 724 to the second aqueous mixture. The ratio of PGA to Methocel HPMC Culminal 724 in the combined aqueous mixture was controlled to be 81/19. The resulting foam was observed to have a wet foam density of about 0.18 g/cc and a dry foam density of about 0.057 g/cc. Figure 9 shows an SEM picture of the foam prepared in this Example 8.

### Example 9

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | None | |
| Non-Surface Active Polymer | Dextran | 1.94 grams |
| Gel Inducing Agent | GDL | 3.77 grams |

The process as described in Example 1 was repeated except the second aqueous mixture was prepared by adding 1.94 grams Dextran to the second aqueous mixture and Methocel HPMC Culminal 724 was omitted from the aqueous mixture. The resulting foam was observed to have a wet foam density of about 0.34 g/cc and a dry foam density of about 0.095 g/cc. The foam was observed to be less porous than the foam formed in Example 1 and the pores were less interconnected than the pores of the foam formed in Example 1. It is believed that the omission of Methocel HPMC Culminal 724, or any polymer having surface activity, reduced the level of interconnectivity between the pores of the foam. Furthermore, the addition of a polymer having no surface activity did not contribute to the formation of pores in the foam. Figure 10 shows an SEM picture of the foam prepared in this Example 9.

### Example 10

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Pluronic^{®} P123 | 1.94 grams |
| Non-Surface Active Polymer | None | |
| Gel Inducing Agent | GDL | 3.77 grams |

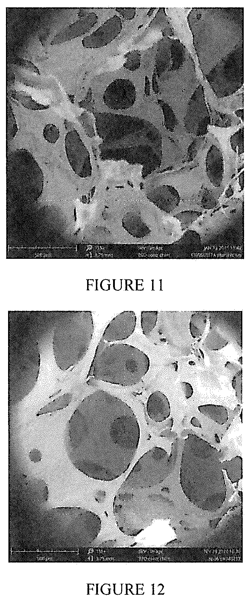

The process as described in Example 1 was repeated except the second aqueous mixture was prepared by adding 1.94 grams Pluronic^{®} P123, instead of Methocel HPMC Culminal 724, to the second aqueous mixture. The ratio of PGA to Pluronic P123 in the combined aqueous mixture was controlled to be 62.5/37.5. The resulting foam was observed to have a wet foam density of about 0.18 g/cc and a dry foam density of about 0.03 g/cc and was observed to have a greater porosity than the foam as formed in Example 1. Figure 11 shows an SEM picture of the foam prepared in this Example 10.

### Example 11

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Pluronic^{®} P123 | 0.97 grams |
| Non-Surface Active Polymer | Dextran | 0.97 grams |
| Gel Inducing Agent | GDL | 3.77 grams |

The process as described in Example 1 was repeated except the second aqueous mixture was prepared by adding a 50:50 weight ratio blend of Pluronic P123 and Dextran instead of Methocel HPMC Culminal 724. The resulting foam was observed to have a wet foam density of about 0.20 g/cc and a dry foam density of about 0.038 g/cc and was observed to have a greater porosity than the foam as formed in Example 1. Figure 12 shows an SEM picture of the foam prepared in this Example 11.

### Example 12

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 6.5 grams; 7.5 grams; 19.44 grams; 29.16 grams |
| Sugar | Sucrose | 17.5 grams |
| Surface Active Polymer | Pluronic^{®} P123 | 0.97 grams |
| Non-Surface Active Polymer | Dextran | 0.97 grams |
| Gel Inducing Agent | GDL | 3.77 grams |

The process as described in Example 11 was repeated to form multiple foams in which varied amounts of glycerol were added to the second aqueous mixture. In forming a first foam of this example, 6.5 grams of glycerol was added the second aqueous mixture. Glycerol constituted 19 wt. % of the total solid additives added to form the combined aqueous mixture. The resulting foam was observed to have a wet foam density of about 0.19 g/cc and a dry foam density of about 0.055 g/cc. In forming a second foam of this example, 7.5 grams of glycerol was added the second aqueous mixture. Glycerol constituted 22 wt. % of the total solid additives added to form the combined aqueous mixture. The resulting foam was observed to have a wet foam density of about 0.21 g/cc and a dry foam density of about 0.048 g/cc. In forming third first foam of this example, 19.44 grams of glycerol was added the second aqueous mixture. Glycerol constituted 42 wt. % of the total solid additives added to form the combined aqueous mixture. The resulting foam was observed to have a wet foam density of about 0.23 g/cc and a dry foam density of about 0.23 g/cc. In forming a fourth foam of this example, 29.16 grams of glycerol was added the second aqueous mixture. Glycerol constituted 52 wt. % of the total solid additives added to form the combined aqueous mixture. The resulting foam was observed to have a wet foam density of about 0.26 g/cc and a dry foam density of about 0.35 g/cc.

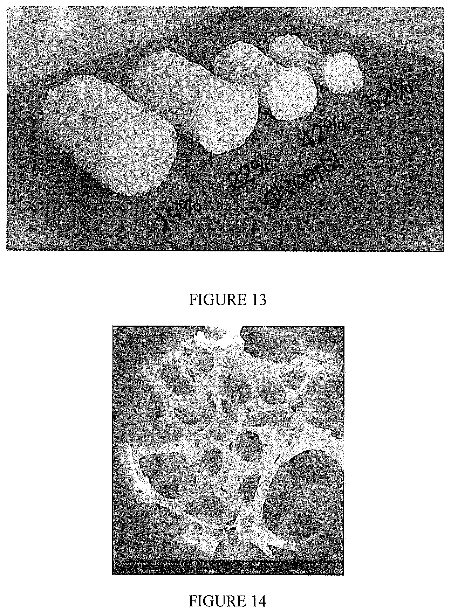

The four foams of Example 12 illustrate the effect of the amount of plasticizer on density and porosity when added during the formation of the foams. Slower drying, greater density and less porosity were observed with increasing plasticizer content. Also, as shown in Figure 13, the generally cylindrical shape of the foam was lost as the amount of glycerol increased above about 20-25 wt. % of the total solid additives added to form the second aqueous mixture. It was observed that porosity and interconnectivity of the pores of the foam was greatest when the amount of plasticizer added to the second aqueous mixture was less than about 52 wt. % of the total solid additives added to form the second aqueous mixture.

### Example 13

| **Additive Component** | **Type of Additive** | **Amount of Additive** |
|---|---|---|
| Polysaccharide | 2.0 wt. % PGA | 162 grams |
| Divalent Metal Salt | CaCO₃ | 1.06 grams |
| Emulsifying Agent | TWEEN^{®} 20 | 0.125 grams |
| Plasticizer | Glycerol | 7.5 grams |
| Sugar | Sucrose | 17.5 grams |
| Surface Active Polymer | Pluronic^{®} P127 | 0.97 grams |
| Non-Surface Active Polymer | Dextran | 0.97 grams |
| Gel Inducing Agent | GDL | 3.77 grams |

The process as described in Example 1 was repeated except the second aqueous mixture was prepared by adding a 50:50 weight ratio blend of Pluronic P127 and Dextran. The resulting foam was observed to have a porosity and interconnectivity of pores similar to the foam prepared in Example 11. Figure 14 shows an SEM picture of the foam prepared in this Example 13.

### Example 14

Foams formed in accordance with each of the processes in Examples 1-12 were coated with Synthemax^{®} II-SC. A 250 µg/ml Synthemax^{®} II-SC aqueous ethanol solution was prepared by adding about 5.0 mg of Corning^{®} Synthemax^{®} II-SC powder to about 20 mL of an aqueous ethanol solution having an ethanol:water ratio of 70:30. Each of the foams, which were about 2-3 mm thick and had diameters of about 22 mm, were placed in separate wells of Polystyrene 6-Well Cell Culture Plates. About 4.0 mL of the 250 µg/ml Synthemax^{®} II-SC aqueous ethanol solution was added to each of the wells and the plates were then left undisturbed at room temperature for about 1.5 hours. Excess solution was removed from the wells and the foams were washed once with about 5.0 mL of a 70% aqueous ethanol. The Synthemax^{®} II-SC coating was then crosslinked by adding about 4.0 mL of a 0.05% v/v glutaraldehyde in 70% aqueous ethanol (prepared by mixing 40µl of 25% glutaraldehyde solution in 6.0 mL water and 14 mL ethanol). The plates were left undisturbed at room temperature for about 1.5 hours to allow time for crosslinking to occur. The foam was then rinsed three times with ultrapure water.

### Example 15

Foams formed in accordance with each of the processes in Examples 1-12 were coated with gelatin. A 0.1 wt. % gelatin solution was prepared by swelling about 500 mg of gelatin powder (from porcine skin) in ultrapure water followed by dissolution and homogenization in 20 mL of ultrapure water. Each of the foams, which were about 2-3 mm thick and had diameters of about 22 mm, were placed in separate wells of Polystyrene 6-Well Cell Culture Plates. About 4.0 mL of the gelatin solution was added to each of the wells and the plates were then left undisturbed at room temperature for about 1.5 hours. Excess solution was removed from the wells and the foams were washed once with about 5.0 mL of a 70% aqueous ethanol. The gelatin coating was then crosslinked by adding about 4.0 mL of a 0.05% v/v glutaraldehyde in 70% aqueous ethanol (prepared by mixing 50µl of 25% glutaraldehyde solution in 25 mL ultrapure water). The plates were left undisturbed at room temperature for about 1.5 hour to allow time for crosslinking to occur. The foam was then rinsed three times with ultrapure water.

### Example 16

Culturing Vero cells on a foam formed in accordance with the process of Example 4 was investigated. Vero cells (ATCC^{®} CCL-81, commercially available from ATCC, Manassas, VA) were cultured on cell culture plates in IMDM medium supplemented with 10% fetal bovine serum (FBS). The foam was cut into portions that were about 2-3 mm thick and had diameters of about 22 mm. The foam portions were sanitized in 70% aqueous ethanol for about 5.0 minutes, then placed in separate wells of a 6-Well Ultra-Low Attachment Cell Culture Plate. The foam portions were washed twice in ultrapure water and once in IMDM medium. Excess medium was removed from the wells before seeding.

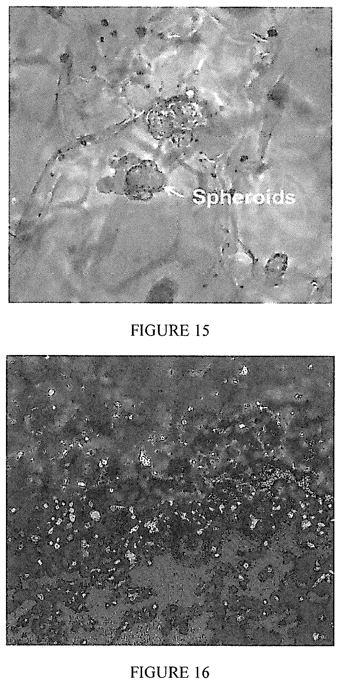

Vero cells were harvested from the cell culture plates using trypsin, re-suspended in IMDM medium and 150 µL containing about 100,000 cells were seeded in each of the foam portions which were positioned in the wells of the 6-Well Cell Culture Plate. The 6- Cell Culture Plate was placed in a cell culture incubator and, after about 2.0 hours, about 3.0 mL of IMDM medium was added to each well. After about 18 hours in the cell culture incubator, the foam portions were visualized using phase contrast microscopy. An image obtained from the phase contrast microscopy is depicted in Figure 15 which shows that the cells did not adhere to the uncoated foam portions, but instead formed spheroids in the pores of the foam portions. As such, it was determined that the dissolvable foam scaffolds of the present disclosure may be utilized to culture spheroids or non-adherent cells.

### Example 17

Culturing Human Mesenchymal Stem Cells (hMSC) on a foam formed in accordance with the process of Example 4 and coated in accordance with the process of Example 14 was investigated. hMSC Passage 2 (commercially available from RoosterBio Inc., Frederick, MD) were cultured on cell culture plates in Mesencult^{™}-XF medium (a serum-free medium commercially available from STEMCELL Technologies, Vancouver, BC, Canada). The foam was cut into portions that were about 2-3 mm thick and had diameters of about 22 mm. The foam portions were sanitized in 70% aqueous ethanol for about 5.0 minutes, then placed in separate wells of a 6-Well Ultra-Low Attachment Cell Culture Plate. The foam portions were washed twice in ultrapure water and once in the Mesencult^{™}-XF medium. Excess medium was removed from the wells before seeding.

hMSC were harvested from the cell culture plates using trypsin, re-suspended in Mesencult^{™}-XF medium and 150 µL containing about 100,000 cells were seeded in each of the foam portions which were positioned in the wells of the 6-Well Cell Culture Plate. The 6-Well Cell Culture Plate was placed in a cell culture incubator and, after about 2.0 hours, about 3.0 mL of Mesencult^{™}-XF medium was added to each well. After about 18 hours in the cell culture incubator, cells were stained with 1 µg/mL Calcein-AM and visualized using fluorescence microscopy. An image obtained from the fluorescence microscopy is depicted in Figure 16 which shows that the cells were able to adhere to the foam portions, and spread within the pores of the foam portion. As such, it was determined that the dissolvable foam scaffolds coated with Corning^{®} Synthemax^{®} II-SC of the present disclosure may be utilized to culture adherent cells in addition to spheroids or non-adherent cells and that the scaffolds support cell culture in serum-free medium.

### Example 18

Culturing Human Mesenchymal Stem Cells (hMSC) on a foam formed in accordance with the process of Example 4 and coated in accordance with the process of Example 15 was investigated. hMSC Passage 2 as used in Example 17 were cultured on cell culture plates in IMDM medium supplemented with 10% fetal bovine serum (FBS). The foam was cut into portions that were about 2-3 mm thick and had diameters of about 22 mm. The foam portions were sanitized in 70% aqueous ethanol for about 5.0 minutes, then placed in separate wells of a 6-Well Ultra-Low Attachment Cell Culture Plate. The foam portions were washed twice in ultrapure water and once in the Mesencult^{™}-XF medium. Excess medium was removed from the wells before seeding.

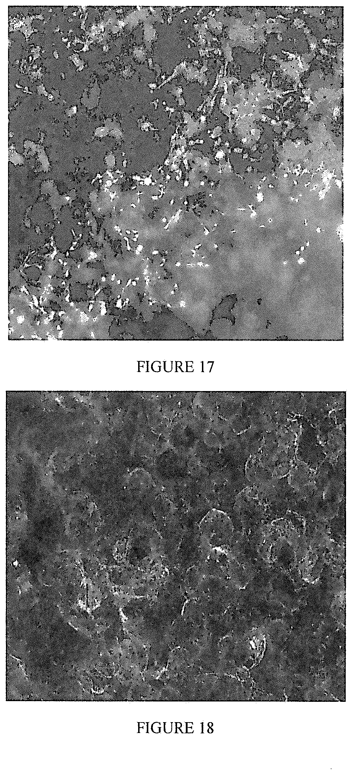

hMSC were harvested from the cell culture plates using trypsin, re-suspended in IMDM medium and 150 µL containing about 100,000 cells were seeded in each of the foam portions which were positioned in the wells of the 6-Well Cell Culture Plate. The 6-Well Cell Culture Plate was placed in a cell culture incubator and, after about 2.0 hours, about 3.0 mL of IMDM medium was added to each well. After about 18 hours in the cell culture incubator, cells were stained with 1µg/mL Calcein-AM and visualized using fluorescence microscopy. An image obtained from the fluorescence microscopy is depicted in Figure 17 which shows that the cells were able to adhere to the foam portions, and spread within the pores of the foam portion. Expansion of the hMSC cells was allowed to proceed on the foam for six days and again visualized using fluorescence microscopy. An image obtained from the fluorescence microscopy after six days is depicted in Figure 18 which shows cells adhered to the foam portions, and further spread within the pores of the foam portion. As such, it was determined that the dissolvable foam scaffolds coated with gelatin of the present disclosure may be utilized to culture adherent cells in addition to spheroids or non-adherent cells and that the scaffolds support cell culture in serum-containing medium.

### Example 19

Expansion of Vero cells on a foam formed in accordance with the processes of Examples 2 and 4 and coated in accordance with the process of Example 15 was investigated. Vero cells as used in Example 16 were cultured on Tissue Culture Treated (TCT) plates in IMDM medium supplemented with 10% fetal bovine serum (FBS). The foam was cut into portions that were about 2-3 mm thick and had diameters of about 22 mm. The foam portions were sanitized in 70% aqueous ethanol for about 5.0 minutes, then placed in separate wells of a Polystyrene 6-Well Cell Culture Plate. The foam portions were washed twice in ultrapure water and once in the IMDM medium. Excess medium was removed from the wells before seeding.

Vero cells were harvested from the TCT plates using trypsin and re-suspended in IMDM medium. Some foam portions were seeded with 150 µL containing about 25,000 cells and other foam portions were seeded with 150 µL containing about 50,000 cells, wherein the foam portions were positioned in the well of the 6-Well Cell Culture Plate. The 6-Well Cell Culture Plate was placed in a cell culture incubator for about 6 days. After about 6 days, the medium was removed and the foams were dissolved by the addition to each of the wells of about 2.0 mL of a digestion solution containing about 50 U/mL pectinase and about 5.0 mM EDTA. The foam was observed to dissolve in under 5.0 minutes. Following dissolution of the foams, cells were counted using the trypan blue exclusion protocol as detailed in "Protocol for Performing a Trypan Blue Viability Test: Technical Reference Guide." Lonza Cologne GmbH, September 2012, retrieved from:

http://bio.lonza.com/uploads/tx_mwaxmarketingmaterial/Lonza_BenchGuides _Protocol_for_Performing_a_Trypan_Blue_Viability_Test_Technical_Reference_Guide.pd f.

It was observed that the cells colonized on all the pore surfaces of the the foam portions. About 70 to 90 fold expansion was observed in the coated foam formed in accordance with the process of Example 4 and about 40 to 70 fold expansion was observed in the coated foam formed in accordance with the process of Example 2. As such, it was determined that the dissolvable foam scaffolds coated with gelatin of the present disclosure may be utilized for the expansion of Vero cells.

### Example 20

Differentiation of Human Mesenchymal Stem Cells (hMSC) on a foam formed in accordance with the process of Example 4 and coated in accordance with the process of Example 15 was investigated. hMSC Passage 2 as used in Example 17 were cultured on cell culture plates in Mesencult^{™}-XF medium. The foam was cut into portions that were about 2-3 mm thick and had diameters of about 22 mm. The foam portions were sanitized in 70% aqueous ethanol for about 5.0 minutes, then placed in separate wells of a Polystyrene 6-Well Cell Culture Plate. The foam portions were washed twice in ultrapure water and once in the Mesencult^{™}-XF medium. Excess medium was removed from the wells before seeding.

hMSC were harvested from the cell culture plates using trypsin, re-suspended in Mesencult^{™}-XF medium and 150 µL containing about 100,000 cells were seeded in each of the foam portions which were positioned in the wells of the 6-Well Cell Culture Plate. The 6-Well Cell Culture Plate was placed in a cell culture incubator for about 3 days. After about 3 days, the Mesencult^{™}-XF medium was removed and osteocyte differentiation medium, chondrocyte differentiation medium, and adipocyte differentiation medium (commercially available under the trade name StemPro^{™} Osteogenesis Differentiation Kit, StemPro^{™} Chondrogenesis Differentiation Kit and StemPro^{™} Adipogenesis Differentiation Kit respectfully from Thermo Fisher Scientific, Waltham, MA) was added to the wells, wherein each type of differentiation medium was added to foam portions in different wells than the other types of differentiation medium. After 3 weeks foam portions exposed to osteocyte differentiation medium were stained with Alizarin Red, foam portions exposed to chondrocyte differentiation medium were stained with Alcian Blue, and foam portions exposed to adipocyte differentiation medium were stained with Oil Red O. After staining, it was observed that hSMC cultured on dissolvable foam scaffolds coated with gelatin of the present disclosure maintain chondrogenic, osteogenic and adipogenic differentiation. As such, it was determined that cells cultured on dissolvable foam scaffolds of the present disclosure exhibit biological responses similar to biological responses of cells in-vivo.

### Example 21

Expansion of Human Mesenchymal Stem Cells (hMSC) on a foam formed in accordance with the process of Examples 2, 11 and 13, and coated in accordance with the process of Example 14 was investigated. hMSC Passage 2 as used in Example 17 were cultured on cell culture plates in Mesencult^{™}-XF medium. The foam was cut into portions that were about 2-3 mm thick and had diameters of about 22 mm. The foam portions were sanitized in 70% aqueous ethanol for about 5.0 minutes, then placed in separate wells of a 6-Well Ultra-Low Attachment Cell Culture Plate. The foam portions were washed twice in ultrapure water and once in the Mesencult^{™}-XF medium. Excess medium was removed from the wells before seeding.

hMSC were harvested from the cell culture plates using trypsin, re-suspended in Mesencult^{™}-XF medium and 150 µL containing about 100,000 cells were seeded in each of the foam portions which were positioned in the wells of the 6-Well Cell Culture Plate. The 6-Well Cell Culture Plate was placed in a cell culture incubator for about 7 days and medium was removed and replaced with fresh medium on day 4. After about 7 days, the medium was removed and the foams were dissolved by the addition to each of the wells of about 2.0 mL of a digestion solution containing about 50 U/mL pectinase and about 5.0 mM EDTA. The foam was observed to dissolve in under 5.0 minutes. Following dissolution of the foams, cells were counted using the trypan blue exclusion protocol previously described. It was observed that cells colonized on all the pore surfaces of the foam portions and that expansion of hSMC on the dissolvable foam scaffolds coated with Corning^{®} Synthemax^{®} II-SC of the present disclosure was successfully accomplished.

### Example 22

Comparison of culturing Human Mesenchymal Stem Cells (hMSC) in static conditions and in dynamic conditions on foams formed in accordance with the processes of Examples 2 and 11, and coated in accordance with the process of Example 14, was investigated. hMSC Passage 2 were cultured as in Example 17. hMSC were harvested from the cell culture plates using trypsin, re-suspended in Mesencult^{™}-XF medium and 150 µL containing about 100,000 cells were seeded in twenty foam scaffolds as formed in Example 2 and in twenty foam scaffolds as formed in Example 11. The scaffolds were located in wells of 6-Well Cell Culture Plates which were placed in a cell culture incubator for about 2 hours. After about 2 hours, a 6-Well Cell Culture Plate containing scaffolds as formed in accordance with Example 2, and a 6-Well Cell Culture Plate containing scaffolds as formed in accordance with Example were removed from the incubator and the scaffolds were transferred to separate bioreactors. The other 6-Well Cell Culture Plates remained in the incubator. All of the scaffolds were kept under their respective conditions for about 6 days and were then dissolved by the addition of a digestion solution containing about 50 U/mL pectinase and about 5.0 mM EDTA. Following dissolution of the foams, cells were counted using the trypan blue exclusion protocol previously described. It was observed that the expansion of cells on the dissolvable foam scaffolds coated with Corning^{®} Synthemax^{®} II-SC of the present disclosure was similar under static conditions as under dynamic conditions.

### Example 23

Multi-passage culturing of Human Mesenchymal Stem Cells (hMSC) was investigated. As described herein, the passage number of a cell culture is a record of the number of times the culture has been subcultured, i.e. harvested and reseeded. Thus, multi-passage culturing in relation to the present disclosure describes a process wherein cells are harvested from one dissolvable foam scaffold and reseeded on a different dissolvable foam scaffold. hMSC were harvested from the cell culture plates using trypsin, re-suspended in Mesencult^{™}-XF medium and 150 µL containing about 100,000 cells were seeded in a first set of foam scaffolds as formed in Example 2 and as coated in Example 14. The scaffolds were placed in Mesencult^{™}-XF medium and placed in a cell culture incubator for about 6 days. After about 6 days, the scaffolds were dissolved by the addition of a digestion solution containing about 50 U/mL pectinase and about 5.0 mM EDTA. Following dissolution of the foams, cells were counted using the trypan blue exclusion protocol previously described. A solution containing about 100,000 cells that were harvested from the first set of scaffolds was then used to seed a second set of foam scaffolds as formed in Example 2 and as coated in Example 14. The scaffolds were placed in Mesencult^{™}-XF medium and placed in a cell culture incubator for about 7 days. After about 7 days, the scaffolds were dissolved by the addition of a digestion solution containing about 50 U/mL pectinase and about 5.0 mM EDTA. Following dissolution of the foams, cells were counted using the trypan blue exclusion protocol. A solution containing about 100,000 cells harvested from the second set of scaffolds was then used to seed a third set of foam scaffolds as formed in Example 2 and as coated in Example 14. The scaffolds were placed in Mesencult^{™}-XF medium and placed in a cell culture incubator for about 7 days. After about 7 days, the scaffolds were dissolved by the addition of a digestion solution containing about 50 U/mL pectinase and about 5.0 mM EDTA. Following dissolution of the foams, cells were counted using the trypan blue exclusion protocol and then seeded on cell culture plates where the cells were exposed to the differentiation medium, the chondrocyte differentiation medium, and the adipocyte differentiation medium used in Example 20. After 3 weeks cells exposed to osteocyte differentiation medium were stained with Alizarin Red, cells exposed to chondrocyte differentiation medium were stained with Alcian Blue, and cells exposed to adipocyte differentiation medium were stained with Oil Red O. After staining, it was observed that hSMC which experienced multi-passage culturing on dissolvable foam scaffolds coated with Corning^{®} Synthemax^{®} II-SC of the present disclosure maintain chondrogenic, osteogenic and adipogenic differentiation.

### Example 24

Transfection of Human embryonic kidney (HEK) cells on a foam scaffold formed in accordance with the process of Example 11 and coated in accordance with the process of Example 14 was compared to the transfection of HEK cells on Corning^{®} Synthemax^{®} II-SC Dissolvable Microcarriers (commercially available from Corning Incorporated, Corning, NY). HEK cells were seeded on the Dissolvable Microcarriers. A first set of Dissolvable Microcarriers were placed in 6-Well Cell Culture Plates and exposed to IMDM medium supplemented with 10% fetal bovine serum (FBS). The 6-Well Cell Culture Plates were placed in a cell incubator and expanded in static conditions for about 3 days. A second set of Dissolvable Microcarriers were placed in a disposable spinner flask and suspended in IMDM medium supplemented with 10% fetal bovine serum (FBS) for about 3 days with intermittent stirring (stir for 15 minutes every 2.0 hours). After about 3 days, about 150 µL of a transfection reagent was added to the Dissolvable Microcarriers in the wells of the 6-Well Cell Culture Plates and about 1.0 mL of a transfection reagent was added to the spinner flask with continued intermittent stirring.

Portions of foam were seeded with HEK cells. Some foam portions were placed in 6-Well Cell Culture Plates, exposed to IMDM medium supplemented with 10% fetal bovine serum (FBS) and then the 6-Well Culture Plates were placed in a cell incubator for about 3 days. Other foam portions were placed in a chamber of a radial flow perfusion cartridge device for about 3 days. The perfusion cartridge device allowed for the continuous removal of spent IMDM medium supplemented with 10% fetal bovine serum (FBS) and addition of fresh IMDM medium supplemented with 10% fetal bovine serum (FBS) from the chamber. After about 3 days, about 150 µL of a transfection reagent was added to the foam portions in the wells of the 6-Well Cell Culture Plates and about 1.0 mL of a transfection reagent was added to the foam portions in the perfusion cartridge device.

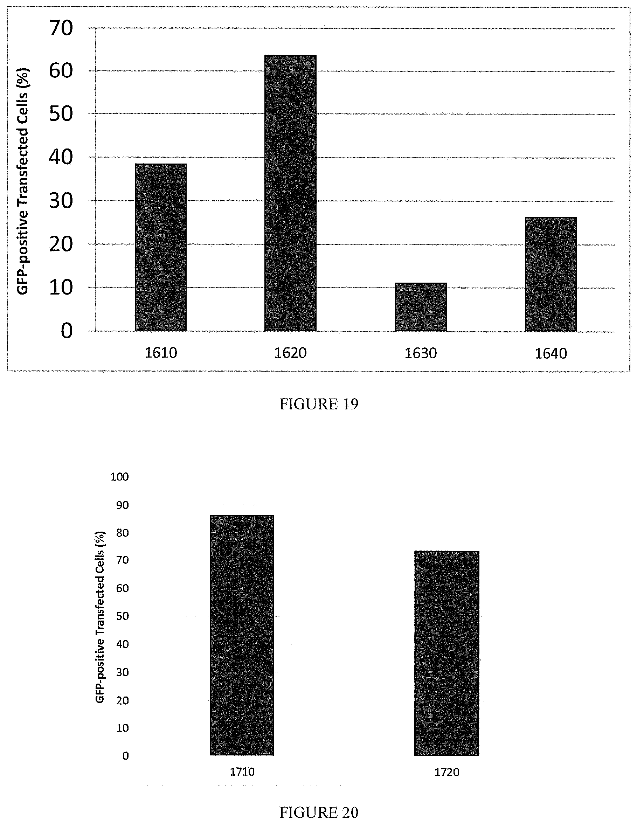

After the addition of the transfection reagent, cells were harvested from the Dissolvable Microcarriers and from the foam portions and reporter gene expression levels were detected by measuring the response of green fluorescent protein (GFP) as reporter genes. GFP-positive percentage of the transfected HEK cells, which may be used as an indicator of transfection efficiency, was measured by flow cytometry. As used herein, the term "transfection efficiency" refers to the percentage of cells that have a given nucleic acid or biologically active molecule present within the cell after exposure to a transfection reagent. Figure 19 is a bar graph showing the GFP-positive percentage of the transfected HEK cells for each of the culture conditions. As shown: bar **1610** represents the GFP-positive percentage of the transfected HEK cells cultured on the dissolvable foams portions in the 6-Well Cell Culture Plates; bar **1620** represents the GFP-positive percentage of the transfected HEK cells cultured on the dissolvable foams portions in the perfusion cartridge device; bar **1630** represents the GFP-positive percentage of the transfected HEK cells cultured on Dissolvable Microcarriers in 6-Well Cell Culture Plates; and bar **1640** represents the GFP-positive percentage of the transfected HEK cells cultured on Dissolvable Microcarriers in the spinner flask. It was observed that cells cultured on dissolvable foam scaffolds coated with Corning^{®} Synthemax^{®} II-SC of the present disclosure exhibited greater transfection efficiency than cells cultured on microcarriers. Without wishing to be bound by any particular theory, it is believed that cells within pores of the foam scaffolds experienced greater exposure to the transfection reagent than cells attached to the surface of a microcarrier.

### Example 25

Production of Adeno-Associated Virus (AAV) vectors on a foam scaffolds formed in accordance with the process of Example 11 and coated in accordance with the process of Example 14 was demonstrated. A first and a second set of foam scaffolds were seeded with 1 million 293aav cells and transferred to a chamber of a radial flow perfusion cartridge device where they were exposed to IMDM medium supplemented with 10% fetal bovine serum (FBS).

After about 24 hours, cells from the first set of foam scaffolds were transfected with an AAV-2 Helper Free Packaging System (commercially available from Cell Biolabs, Inc., San Diego, CA) using a Calcium Phosphate transfection method. Also after about 24 hours, cells from the second set of foam scaffolds were transfected with an AAV-2 Helper Free Packaging System using a PEI method. For the Calcium Phosphate method, cells were incubated for about 18 hours with calcium phosphate/ DNA complexes prepared with a plasmid concentration of 6.4 µg/mL and a 1:1:1 molar ratio of plasmids. For the PEI method, cells on the foam scaffolds were transfected with 2µg plasmid/mL. In each of the PEI methods, PEI/DNA ratios of 2/1 and 1: 1: 1 molar ratio of plasmids were used.

The radial flow perfusion cartridge devices were operated at a perfusion rate of 20 mL/min for about 2 hours and then at 5 mL/min for about 16 hours. For all sets of cells, medium was removed and replaced with fresh medium after about 18 hours. Cells were then incubated further and harvested 72 hours after transfection. The foam scaffolds were collected and dissolved by the addition of a digestion solution containing about 50 U/mL pectinase and about 5.0 mM EDTA. Following dissolution of the foam scaffolds, cells were collected using centrifugation and washed in dPBS. A portion of the cells were kept for flow cytometry analysis, and the remaining cells were lyzed using a 20mM tris pH8, 150mM NaCl, 0.1% sodium deoxycholate buffer supplemented with 50U/mL benzonase.

After about 30 minutes of incubation at 37°C, viral extracts were clarified by centrifugation for about 5 min at 14,000 rpm. Viral titers were measured using the AAV-2 ELISA assay (commercially available from PROGEN Biotechnik GmbH, Heidelberg, Germany).

Transfection efficiency was analyzed using flow cytometry and good transfection efficiency was observed. Figure 20 is a bar graph showing the GFP-positive percentage of the transfected cells for each of the sets of foam scaffolds. As shown: bar **1710** represents the GFP-positive percentage of the cells transfected using the Calcium Phosphate transfection method in which transfection method in which transfection efficiency was measured to be about 86.4%; and bar **1720** represents the GFP-positive percentage of the cells transfected using the PEI transfection method in which transfection efficiency was measured to be about 73.8%.

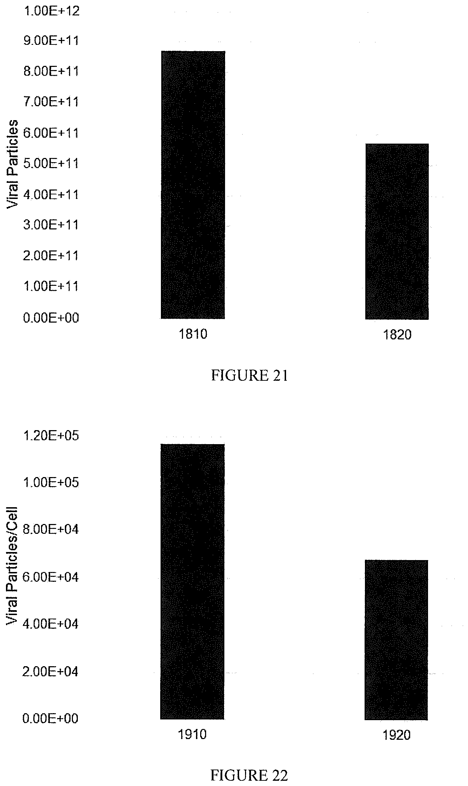

Virus titer was also measured using ELISA assay. Figure 21 is a bar graph showing the number of viral particles (vp) per set of foam scaffold. As shown: bar **1810** represents the viral particles of the cells transfected using the Calcium Phosphate transfection method in which 8.7 x 10¹¹ viral particles were measured; and bar **1820** represents viral particles of the cells transfected using the PEI transfection method in which 5.7 x 10¹¹ viral particles were measured. Figure 22 is a bar graph showing the number of viral particles per cell per set of foam scaffold. As shown: bar **1910** represents the viral particles/cell of the cells transfected using the Calcium Phosphate transfection method in which 1.17 x 10⁵ viral particles/cell were measured; and bar **1920** represents viral particles/cell of the cells transfected using the PEI transfection method in which 6.8 x 10⁴ viral particles/cell were measured.

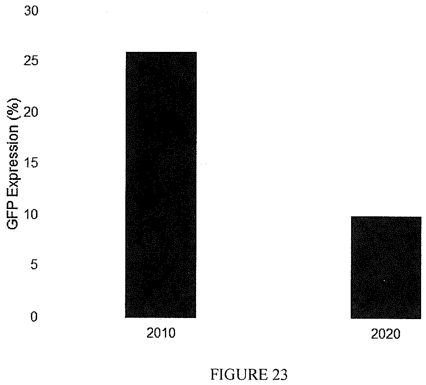

Finally, the functionality of the viral vectors was assessed by testing the extracts capacity to induce GFP expression in the infected HEK 293 cells. Viral vector infectivity was assessed by infecting HEK 293 cells with extracts prepared using 10⁵ viral particles/cell to infect. About 72 hours after infection, GFP-positive percentage of the infected HEK cells was measured by flow cytometry. Infection was observed for cells from each of the sets of foam scaffolds. GFP expression was used to identify the efficiency of gene transfer, which for the present example ranged from about 10% to about 26%. As such, it was concluded that functional AAV vectors can be produced on foam scaffolds as disclosed herein. Figure 23 is a bar graph showing the fraction of cells exhibiting GFP expression for each of the sets of foam scaffolds after infection. As shown: bar **2010** represents the fraction of cells exhibiting GFP expression for the cells transfected using the Calcium Phosphate transfection method in which about 26% of the cells exhibited GFP expression; and bar **2020** represents the fraction of cells exhibiting GFP expression for the cells transfected using the PEI transfection method in which about 10% of the cells showed GFP expression.

While the present disclosure includes a limited number of embodiments, those skilled in the art, having benefit of this disclosure, will appreciate that other embodiments can be devised which do not depart from the scope of the present disclosure.

## Claims

1. A dissolvable foam scaffold for cell culture comprising:
an ionotropically crosslinked polygalacturonic acid compound selected from at least one of: pectic acid; partially esterified pectic acid, partially amidated pectic acid and salts thereof;
at least one first water-soluble polymer having surface activity, the at least one first water-soluble polymer being selected from the group consisting of hydroxyethylcellulose (HEC), hydroxypropylcellulose (HPC), methylcellulose (MC), hydroxyethylmethylcellulose (HEMC), hydroxypropyl-methylcellulose (HPMC), bovine serum albumin (BSA), gelatine, casein, hydrophobins, and poloxamer;
at least one second polymer having no surface activity, the at least one second polymer comprising dextran; and
a water-soluble plasticizer, wherein the dissolvable foam scaffold comprises between 15 wt.% and 55 wt. % water-soluble plasticizer, and
wherein the dissolvable foam scaffold has a dry density of less than 0.11 g/cc.

2. The dissolvable foam scaffold of claim 1 further comprising an adhesion polymer coating.

3. The dissolvable foam scaffold of claim 2,
wherein the adhesion polymer coating comprises peptides;
wherein the adhesion polymer coating comprises peptides selected from the group consisting of BSP, vitronectin, fibronectin, laminin, Type I collagen, Type IV collagen, denatured collagen and mixtures thereof; or
wherein the adhesion polymer coating comprises an RGD peptide-containing copolymer.

4. The dissolvable foam scaffold of any of the preceding claims, wherein the at least one first polymer has a hydrophilic-lipophilic balance (HLB) of greater than 8.

5. The dissolvable foam scaffold of any of the preceding claims comprising at least two first water-soluble polymers having surface activity.

6. The dissolvable foam scaffold of any of the preceding claims wherein the at least one second polymer comprises a synthetic polymer, a semisynthetic polymer, or a natural polymer.

7. The dissolvable foam scaffold of any of the preceding claims comprising less than 50 wt. % water-soluble plasticizer.

8. The dissolvable foam scaffold of any of the preceding claims comprising an average pore size diameter of between 50 µm and 500 µm.

9. A method for forming a dissolvable foam scaffold, the method comprising:
forming a first aqueous mixture by adding a polygalacturonic acid compound selected from at least one of: pectic acid; partially esterified pectic acid, partially amidated pectic acid and salts thereof to an aqueous solution;
forming a second aqueous mixture by adding at least one first water-soluble polymer having surface activity and a divalent metal salt to an aqueous solution;
combining the first aqueous mixture with the second aqueous mixture to form a combined aqueous mixture;
adding a gel inducing agent to the combined aqueous mixture; and
introducing gas bubbles into the combined aqueous mixture to form a foam scaffold,
wherein the at least one first water-soluble polymer being selected from the group consisting of hydroxyethylcellulose (HEC), hydroxypropylcellulose (HPC), methylcellulose (MC), hydroxyethylmethylcellulose (HEMC), hydroxypropyl-methylcellulose (HPMC), bovine serum albumin (BSA), gelatine, casein, hydrophobins, and poloxamer, and
wherein forming a second aqueous mixture further comprises adding at least one second polymer having no surface activity and a water-soluble plasticizer, the at least one second polymer comprising dextran, and
wherein the foam scaffold comprises between 15 wt.% and 55 wt. % of the water-soluble plasticizer, and
wherein the dissolvable foam scaffold has a dry density of less than 0.11 g/cc.

10. The method of claim 9, wherein forming a second aqueous mixture further comprises adding at least two first water-soluble polymers having surface activity.

11. The method of any of claims 9-10, wherein the divalent metal salt comprises:
a cation selected from the group consisting of magnesium, calcium, zinc, strontium, barium, and combinations thereof; and
an anion selected from the group consisting of oxalates, tartrates, phosphates, carbonates, citrates, and combinations thereof.

12. The method of any of claims 9-11, wherein the water-soluble plasticizer comprises less than 50 wt. % of the total solid additives of the second aqueous mixture.

13. The method of any of claims 9-12, further comprising coating the dissolvable foam scaffold with an adhesion polymer coating, wherein the adhesion polymer coating optionally comprises peptides.

14. The dissolvable foam scaffold of any of claims 1-8, wherein the dissolvable foam scaffold comprises equal parts by weight of the at least one first water-soluble polymer and the at least one second polymer.

15. The method of any of claims 9-13, wherein the second aqueous mixture comprises equal parts by weigh of the at least one first water-soluble polymer and the at least one second polymer.

## Patentansprüche

1. Lösliches Schaumgerüst für Zellkultur, umfassend:
eine ionotrop vernetzte Polygalacturonsäureverbindung ausgewählt aus zumindest einem von: Pektinsäure; teilweise veresterter Pektinsäure, teilweise amidierter Pektinsäure und Salzen davon;
zumindest ein erstes wasserlösliches Polymer mit Oberflächenaktivität, wobei das zumindest eine erste wasserlösliche Polymer ausgewählt ist aus der Gruppe bestehend aus Hydroxyethylcellulose (HEC), Hydroxypropylcellulose (HPC), Methylcellulose (MC), Hydroxyethylmethylcellulose (HEMC), Hydroxypropylmethylcellulose (HPMC), Rinderserumalbumin (BSA), Gelatine, Casein, Hydrophobinen und Poloxamer;
zumindest ein zweites Polymer, das keine Oberflächenaktivität aufweist, wobei das zumindest eine zweite Polymer Dextran umfasst; und
einen wasserlöslichen Weichmacher, wobei das lösliche Schaumgerüst zwischen 15 Gew.-% und 55 Gew.-% wasserlöslichen Weichmacher umfasst, und
wobei das lösliche Schaumgerüst eine Trockendichte von weniger als 0,11 g/cc aufweist.

2. Lösliches Schaumgerüst nach Anspruch 1, ferner umfassend eine Haftpolymerbeschichtung.

3. Lösliches Schaumgerüst nach Anspruch 2,
wobei die Haftpolymerbeschichtung Peptide umfasst;
wobei die Haftpolymerbeschichtung Peptide umfasst, die ausgewählt sind aus der Gruppe bestehend aus BSP, Vitronektin, Fibronektin, Laminin, Kollagen Typ I, Kollagen Typ IV, denaturiertem Kollagen und Mischungen davon; oder
wobei die Haftpolymerbeschichtung ein RGD-Peptid enthaltendes Copolymer umfasst.

4. Lösliches Schaumgerüst nach einem der vorhergehenden Ansprüche, wobei das zumindest eine erste Polymer ein hydrophiles-lipophiles Gleichgewicht (HLB) von größer als 8 aufweist.

5. Lösliches Schaumgerüst nach einem der vorhergehenden Ansprüche, umfassend zumindest zwei erste wasserlösliche Polymere mit Oberflächenaktivität.

6. Lösliches Schaumgerüst nach einem der vorhergehenden Ansprüche, wobei das zumindest eine zweite Polymer ein synthetisches Polymer, ein halbsynthetisches Polymer oder ein natürliches Polymer umfasst.

7. Lösliches Schaumgerüst nach einem der vorhergehenden Ansprüche, umfassend weniger als 50 Gew.-% wasserlöslichen Weichmacher.

8. Lösliches Schaumgerüst nach einem der vorhergehenden Ansprüche, umfassend einen durchschnittlichen Porengrößendurchmesser zwischen 50 µm und 500 µm.

9. Verfahren zum Bilden eines löslichen Schaumgerüsts, wobei das Verfahren Folgendes umfasst:
Bilden einer ersten wässrigen Mischung durch Hinzufügen einer Polygalacturonsäureverbindung ausgewählt aus zumindest einem von: Pektinsäure; teilweise veresterter Pektinsäure, teilweise amidierter Pektinsäure und Salzen davon zu einer wässrigen Lösung;
Bilden einer zweiten wässrigen Mischung durch Hinzufügen von zumindest einem ersten wasserlöslichen Polymer mit Oberflächenaktivität und einem zweiwertigen Metallsalz zu einer wässrigen Lösung;
Kombinieren der ersten wässrigen Mischung mit der zweiten wässriger Mischung, um eine kombinierte wässrige Mischung zu bilden;
Hinzufügen eines Gelbildungsmittels zu der kombinierten wässrigen Mischung; und
Einbringen von Gasblasen in die kombinierte wässrige Mischung, um ein Schaumgerüst zu bilden,
wobei das zumindest eine erste wasserlösliche Polymer ausgewählt ist aus der Gruppe bestehend aus Hydroxyethylcellulose (HEC), Hydroxypropylcellulose (HPC), Methylcellulose (MC), Hydroxyethylmethylcellulose (HEMC), Hydroxypropylmethylcellulose (HPMC), Rinderserumalbumin (BSA), Gelatine, Casein, Hydrophobinen und Poloxamer, und
wobei das Bilden einer zweiten wässrigen Mischung ferner Hinzufügen von zumindest einem zweiten Polymer, das keine Oberflächenaktivität aufweist, und einem wasserlöslichen Weichmacher umfasst, wobei das zumindest eine zweite Polymer Dextran umfasst, und
wobei das Schaumgerüst zwischen 15 Gew.-% und 55 Gew.-% des wasserlöslichen Weichmachers umfasst, und
wobei das lösliche Schaumgerüst eine Trockendichte von weniger als 0,11 g/cc aufweist.

10. Verfahren nach Anspruch 9, wobei das Bilden einer zweiten wässrigen Mischung ferner Hinzufügen von zumindest zwei ersten wasserlöslichen Polymeren mit Oberflächenaktivität umfasst.

11. Verfahren nach einem der Ansprüche 9-10, wobei das zweiwertige Metallsalz Folgendes umfasst:
ein Kation ausgewählt aus der Gruppe bestehend aus Magnesium, Calcium, Zink, Strontium, Barium und Kombinationen davon; und
ein Anion ausgewählt aus der Gruppe bestehend aus Oxalaten, Tartraten, Phosphaten, Carbonaten, Citraten und Kombinationen davon.

12. Verfahren nach einem der Ansprüche 9-11, wobei der wasserlösliche Weichmacher weniger als 50 Gew.-% der gesamten festen Additive der zweiten wässrigen Mischung umfasst.

13. Verfahren nach einem der Ansprüche 9-12, ferner umfassend Beschichten des löslichen Schaumgerüsts mit einer Haftpolymerbeschichtung, wobei die Haftpolymerbeschichtung optional Peptide umfasst.

14. Lösliches Schaumgerüst nach einem der Ansprüche 1-8, wobei das lösliche Schaumgerüst gleiche Gewichtsteile des zumindest einen ersten wasserlöslichen Polymers und des zumindest einen zweiten Polymers umfasst.

15. Verfahren nach einem der Ansprüche 9-13, wobei die zweite wässrige Mischung gleiche Gewichtsteile des zumindest einen ersten wasserlöslichen Polymers und des zumindest einen zweiten Polymers umfasst.

## Revendications

1. Échafaudage soluble de mousse pour la culture cellulaire comprenant :
un composé acide polygalacturonique réticulé de manière ionotropique choisi parmi au moins un des : acide pectique, acide pectique partiellement estérifié, acide pectique partiellement amidé et des sels de ceux-ci ;
au moins un premier polymère hydrosoluble comportant une activité de surface, le ou les premiers polymères hydrosolubles ayant été choisis parmi le groupe constitué d'hydroxyéthylcellulose (HEC), hydroxypropylcellulose (HPC), méthylcellulose (MC), hydroxyéthylméthylcellulose (HEMC), hydroxypropylméthylcellulose (HPMC), albumine sérique bovine (BSA), gélatine, caséine, hydrophobines et poloxamère ;
au moins un second polymère ne comportant pas d'activité de surface, le ou les seconds polymères comprenant du dextrane ; et
un plastifiant hydrosoluble, l'échafaudage soluble de mousse comprenant entre 15 % en poids et 55 % en poids de plastifiant hydrosoluble, et
l'échafaudage soluble de mousse comportant une densité à sec inférieure à 0,11 g/cc.

2. Échafaudage soluble de mousse de la revendication 1 comprenant en outre un revêtement de polymère d'adhérence.

3. Échafaudage soluble de mousse de la revendication 2,
dans lequel le revêtement de polymère d'adhérence comprend des peptides ;
dans lequel le revêtement de polymère d'adhérence comprend des peptides choisis parmi le groupe constitué de BSP, vitronectine, fibronectine, laminine, collagène de type I, collagène de type IV, collagène dénaturé et des mélanges de ceux-ci ; ou
dans lequel le revêtement de polymère d'adhérence comprend un copolymère contenant un peptide RGD.

4. Échafaudage soluble de mousse de l'une quelconque des revendications précédentes, dans lequel le ou les premiers polymères comportent un équilibre hydrophile-lipophile (HLB) supérieur à 8.

5. Échafaudage soluble de mousse de l'une quelconque des revendications précédentes comprenant au moins deux premiers polymères hydrosolubles comportant une activité de surface.

6. Échafaudage soluble de mousse de l'une quelconque des revendications précédentes dans lequel le ou les seconds polymères comprennent un polymère synthétique, un polymère semi-synthétique ou un polymère naturel.

7. Échafaudage soluble de mousse de l'une quelconque des revendications précédentes comprenant moins de 50 % en poids de plastifiant soluble à l'eau.

8. Échafaudage soluble de mousse de l'une quelconque des revendications précédentes, comprenant un diamètre moyen des pores compris entre 50 µm et 500 µm.

9. Procédé de formation d'un échafaudage soluble de mousse, le procédé comprenant :
la formation d'un premier mélange aqueux en ajoutant un composé acide polygalacturonique choisi parmi au moins un des : acide pectique ; acide pectique partiellement estérifié, acide pectique partiellement amidé et des sels de ceux-ci à une solution aqueuse ;
la formation d'un second mélange aqueux en ajoutant au moins un premier polymère hydrosoluble comportant une activité de surface et un sel métallique divalent à une solution aqueuse ;
la combinaison du premier mélange aqueux avec le second mélange aqueux pour former un mélange aqueux combiné ;
l'ajout d'un agent gélifiant au mélange aqueux combiné ; et
l'introduction de bulles de gaz dans le mélange aqueux combiné pour former un échafaudage de mousse,
dans lequel le ou les premiers polymères hydrosolubles sont choisis parmi le groupe constitué d'hydroxyéthylcellulose (HEC), hydroxypropylcellulose (HPC), méthylcellulose (MC), hydroxyéthylméthylcellulose (HEMC), hydroxypropylméthylcellulose (HPMC), albumine sérique bovine (BSA), gélatine, caséine, hydrophobines, et poloxamère, et
dans lequel la formation d'un second mélange aqueux comprend en outre l'ajout d'au moins un second polymère ne comportant aucune activité de surface et d'un plastifiant soluble dans l'eau, le ou les second polymères comprenant du dextrane, et
dans lequel l'échafaudage de mousse comprend entre 15 % en poids et 55 % en poids du plastifiant hydrosoluble, et
dans lequel l'échafaudage soluble en mousse a une densité à sec inférieure à 0,11 g/cc.

10. Procédé de la revendication 9, dans lequel la formation d'un second mélange aqueux comprend en outre l'ajout d'au moins deux premiers polymères hydrosolubles comportant une activité de surface.

11. Procédé de l'une quelconque des revendications 9 à 10, dans lequel le sel métallique divalent comprend :
un cation choisi parmi le groupe constitué de magnésium, calcium, zinc, strontium, baryum et de combinaisons de ceux-ci ; et
un anion choisi parmi le groupe constitué d'oxalates, tartrates, phosphates, carbonates, citrates et de combinaisons de ceux-ci.

12. Procédé de l'une quelconque des revendications 9 à 11, dans lequel le plastifiant hydrosoluble comprend moins de 50 % en poids des additifs solides totaux du second mélange aqueux.

13. Procédé de l'une quelconque des revendications 9 à 12, comprenant en outre le revêtement de l'échafaudage soluble de mousse avec un revêtement de polymère d'adhérence, dans lequel le revêtement de polymère d'adhérence comprend éventuellement des peptides.

14. Échafaudage soluble de mousse selon l'une quelconque des revendications 1 à 8, l'échafaudage soluble de mousse comprenant des parties en poids égales du ou des premiers polymères hydrosolubles et du ou des seconds polymères.

15. Procédé de l'une quelconque des revendications 9 à 13, dans lequel le second mélange aqueux comprend des parties en poids égales du ou des premiers polymères hydrosolubles et du ou des seconds polymères.
